# EUROPEAN PATENT SPECIFICATION

(11) **EP 1 431 746 B1**
(45) Date of publication and mention of the grant of the patent: **14.03.2012**
(21) Application number: 02714468.2
(22) Date of filing: 04.04.2002
(51) Int. Cl.: G01N 17/00, A24C 5/32, C12M 3/00

(54) **Entire smoke exposure device**
Ganzrauchaussetzungseinrichtung
Dispositif d'exposition a la fumée

(30) Priority: 28.09.2001 JP 2001303668
(43) Date of publication of application: 23.06.2004
(73) Proprietor: Japan Tobacco Inc., Tokyo 105-8422 (JP)
(72) Inventor: FUKANO, Yasuo, JAPAN TOBACCO INC., Yokohama-shi, Kanagawa 227-0052 (JP); SUZUKI, Mutsuaki, JAPAN TOBACCO INC., Yokohama-shi, Kanagawa 227-0052 (JP); IDE, Maiko, JAPAN TOBACCO INC., Yokohama-shi, Kanagawa 227-0052 (JP)
(74) Representative: Isarpatent
(86) International application number: PCT/JP2002/003399
(87) International publication number: WO 2003/031946

(56) References cited:
- DE-A- 19 526 533
- BOMBICK D.W. ET AL.: 'Chemical and biological studies of a new cigarette that primarily heats tobacco. Part 3. In vitro toxicity of whole smoke' FOOD AND CHEMICAL TOXICOLOGY vol. 36, no. 3, March 1998, pages 191 - 197, XP002954155

## Description

### Technical Field

The present invention relates to an apparatus for exposing cells to smoke containing particulate matter. This apparatus is aimed for exposure tests of cells to aerosols such as tobacco smoke. However, the apparatus is not limited to them and may be applied to exposure tests using other samples.

### Background Art

As apparatuses for exposing cultured cells to samples such as smoke and checking their influences on the cells, a Fraunhofer research institute's exposure apparatus disclosed in Internal Publication Number WO99/36505 and R. J. Reynolds's exposure apparatus (Food and Chemical Toxicology 36 (1998) 191 - 197) are known.

In the Fraunhofer research institute's exposure apparatus, clean air is made to always flow into a chamber. The smoke emitted by a smoke emitting section is introduced into the chamber through the flow of clean air only when blown, and is brought into contact with a cell in the chamber, thereby exposing the cell to the smoke.

In the Reynolds's exposure apparatus, the sample emitted by a smoke emitting section is temporarily retained in a vessel to set a concentration for the sample. The sample whose concentration has been set is always supplied from this vessel into the chamber. The sample is continuously introduced into and exhausted from the chamber. That is, the sample is brought into contact with a cell surface through a continuous flow into the non-sealed chamber, thereby exposing the cell surface to the sample.

These apparatuses differ in that cell surfaces are exposed to smoke containing particulate matter continuously or intermittently, but are common in that cell surfaces are exposed to a sample by using an exposure principle of bringing a sample into contact with the cell surfaces through the continuous flow of air. These apparatuses are effective when a sample has a vapor phase.

According to the exposure principle of bringing the sample into contact with the cell surface through the continuous flow of air in exposing a cell surface to a sample, the cell surface may not be sufficiently exposed to smoke containing a particulate phase.

According to the exposure principle for a conventional apparatus, cell surfaces can be sufficiently exposed to a sample in a vapor phase, on the basis of the diffusion principle. However, a sample in a particulate phase is exhausted through the flow of air continuously flowing in a non-sealed chamber because of the size of particles. This causes a considerable reduction in exposure efficiency of exposure of cells to a particulate phase portion in the sample. In an exposure test conducted by an exposure apparatus having the above exposure principle, smoke containing a particulate phase varies in the exposure ratios of constituents in smoke as compared with a sample in a vapor phase. The test result may not reflect the true influence on the sample. In an exposure test of smoke containing a particulate phase using an apparatus having the conventional exposure principle, a cell surface is not properly exposed to an aerosol amount with a set concentration.

Document DE 195 26 533 A1 describes a device for exposing life cell cultures to gas mixtures with gas- and/or particle-like additives, wherein the gas mixture which is enriched in a defined way is lead to at least one carrier for the life cell cultures by means of a pipeline system, wherein the culture medium can be lead to the life cell cultures from below through a permeable membrane by means of a further pipeline system, and wherein the pipeline system for the enriched gas mixture is configured such that it acts exposing on cell cultures from above.

It is therefore hoped to develop an exposure apparatus having a principle of reliably exposing a cell to a sample such as smoke containing a particulate phase, e.g., tobacco smoke, in particular.

### Disclosure of Invention

It is an object of the present invention to provide a whole smoke exposure apparatus which allows cells to be sufficiently and efficiently exposed to smoke even in an exposure test using smoke containing a particulate phase and/or a vapor phase as a sample.

In order to solve the above problems, according to the present invention, there is provided a whole smoke exposure apparatus comprising a smoke emitting section which emits smoke containing particulate matter; a dilution means for diluting the smoke emitted by the smoke emitting section, by adding a gas to the smoke; an air unit which supplies the gas to said dilution means; at least one exposure chamber which has a cell-adhered surface therein and into which the smoke diluted by the dilution means is introduced; a blocking means for shutting off a gas flow so as to seal the chamber after the smoke is introduced into the chamber a culture medium supply tank, a tube pump, a pipe line connecting the culture medium supply tank to the tube pump and a pipe line connecting the tube pump to the at least one exposure chamber for supplying a culture medium in said at least one exposure chamber so that the cell-adhered surface is immersed in the culture medium to protect a cell attached to the cell-adhered surface when the smoke is introduced into the exposure chamber; a culture medium discharge tank, a discharge tube pump, a pipe line connecting the culture medium discharge tank to the discharge tube pump and a discharge pipe line connecting the discharge tube pump to the at least one exposure chamber for lowering a level of the culture medium lower than the cell-adhered surface after the smoke is introduced into the chamber, and an exhaust means for exhausting the smoke introduced into the chamber. Preferably, the dilution means includes concentration setting means for arbitrarily setting a concentration for a smoke to be diluted.

According to this arrangement, since the flow of smoke into the chamber is blocked at the time of exposure, smoke with a set concentration is brought into contact with a cell-adhered surface in a sealed state without changing the set concentration, thereby efficiently exposing the cell-adhered surface to the smoke. In the chamber into which the flow of smoke is blocked, since a particulate phase in the sample settles down in the chamber for a predetermined period of time, a cell can be reliably exposed to even smoke containing a particulate phase as well as a vapor phase. In addition, if the dilution means has a concentration setting means for arbitrarily setting a dilution concentration, a predetermined amount of sample can be introduced into the chamber.

The whole smoke exposure apparatus further comprises a header which is located between the smoke emitting section and the chamber and in which smoke emitted by the smoke emitting section is temporarily stored before being introduced into the chamber. Preferably, the apparatus includes a three-way connector having one pipe line connected to the header, another pipe line connected to the exhaust means, and still another pipe line open to the atmosphere.

This arrangement is designed to store the sample emitted by the smoke emitting section in the header so as to dilute the sample instead of making a gas always flow in the chamber. Therefore, the sample is not swept away by the continuous flow of a gas, and hence a loss in the absolute amount of sample can be suppressed when the sample is diluted. This also contributes to a reduction in pressure fluctuations at the time of generation of a sample and stabilization of secondary dilution. Furthermore, when smoke in the header is exhausted, an abrupt pressure fluctuation in the header can be prevented by the three-way connector. The sample emitted by the smoke emitting section may be diluted in both the front and rear portions of the header.

The whole smoke exposure apparatus may include a manifold which is positioned between the smoke emitting section and a plurality of chambers for exposure, and has a piping structure uniformly branched to allow smoke emitted by the smoke emitting section to be introduced to be supplied to the plurality of chamber for exposure. Alternatively, the apparatus may include a dilution tank which is positioned between the smoke emitting section and the plurality of chambers for exposure to allow smoke diluted by the dilution means to flow into the dilution tank, and has a piping structure uniformly connected to the plurality of chambers for exposure.

According to this arrangement, samples uniform in concentration and amount can be delivered into a plurality of chambers as compared with a case wherein samples are directly delivered from the dilution means into the respective chambers through glass pipes or the like. In the whole smoke exposure apparatus having a plurality of chambers, a high-precision exposure test can be conducted, and the exposure efficiency improves.

The combination, number, and layout order of constituent elements of the header, manifold, and dilution tank can be arbitrarily changed between the smoke emitting section and the chamber as long as the smoke can be efficiency introduced into the chamber. As an example of the structure of the whole smoke exposure apparatus having these constituent elements, a structure can be conceived which has the smoke emitting section, header, dilution means, and dilution tank arranged in the order named. Another example can be conceived which has the smoke emitting section, header, manifold, and dilution means arranged in the order named (in this case, no dilution tank is provided).

The chamber has an inlet for the smoke which is positioned at an upper portion of the chamber, and an outlet for the smoke which is positioned near the cell-adhered surface.

As a consequence, after settling down/exposure, the sample is efficiently exhausted from near the cell-adhered surface.

The chamber has a structure which has an inlet and outlet for a culture medium in which the cell-adhered surface is to be immersed, and in which the culture medium inlet and outlet are provided at different positions.

According to the above arrangement, the chamber has a structure having a culture medium inlet and outlet independently formed. As a consequence, the culture medium in which a soluble component in a sample dissolves owing to exposure is discharged from the outlet, and a new culture medium is supplied from the inlet different from the outlet. This makes it possible to prevent a culture medium containing unnecessary substances from mixing with a fresh culture medium. In the whole smoke exposure apparatus having this arrangement, a new culture medium is always supplied into the chamber every time an exposure test is conducted. Therefore, this apparatus is also suitable for consecutive exposure tests to be conducted while culture media are replaced. If there are a plurality of chambers identical to the above chamber, each chamber may have a culture medium inlet and outlet. The replacement speed of culture media is preferably set to a predetermined speed enough to prevent any adverse effect on cells.

The positions of the culture medium inlet and outlet can be so changed as to improve the culture medium replacement efficiency in accordance with the state in the chamber. For example, one of the inlet and outlet may be position above the other. Preferably, the inlet and outlet have the same arrangement, so that their functions can be interchanged.

It is preferable that the shape of the chamber be optimally designed to improve the culture medium replacement efficiency. If, for example, the culture medium inlet is positioned above the outlet, the lower portion of the chamber may taper off toward the outlet. According to this shape, since a culture medium automatically gathers at the outlet, the culture medium can be efficiently replaced with a new culture medium. The amount of culture medium required can therefore be reduced. In addition, this can eliminate the fear that soluble components affect a cell.

Preferably, the cell-adhered surface has at least one hole through which the culture medium passes.

According to this arrangement, the fluid level of a culture medium can be quickly lowered to a level lower than the cell-adhered surface so as to bring the cell-adhered surface into contact with a sample at the time of exposure, and can be quickly raised to a level higher than the cell-adhered surface so as to protect the cells after exposure. This makes it possible to raise and lower the fluid level of a culture medium in the chamber even during consecutive exposure tests in which a sample is introduced into the chamber, exhausted after exposure, and introduced again into the chamber. The whole smoke exposure apparatus is also suitable for consecutive exposure tests in which the fluid level of a culture medium is raised and lowered. The speed at which the fluid level of a culture medium is raised and lowered is preferably set to a predetermined speed high enough to prevent any adverse effect on cells.

The whole smoke exposure apparatus may have a means for controlling the fluid level of a culture medium so as to raise and lower it with respect to a cell-adhered surface in the chamber.

It is preferable that the whole smoke exposure apparatus further comprise warm keeping means for keeping the culture medium and the cell-adhered surface at a constant temperature.

As this warm keeping means, for example, a circulating device which keeps a constant temperature by circulating an isothermal medium can be used. Preferably, this temperature to be kept is 35°C to 40°C.

Preferably, the whole smoke exposure apparatus further comprises means for measuring and controlling a pressure in the chamber.

According to this arrangement, when a pressure change occurs in each constituent element in operation, the influence of the pressure change on cells can be avoided by controlling the pressure in the chamber. This pressure control can be realized by, for example, opening/closing a valve provided in each pipe line connected to the chamber in accordance with pressure fluctuations.

More preferably, the whole smoke exposure apparatus further comprises at least one monitor chamber which has a concentration measurement sensor for measuring a concentration of smoke in the chamber at the time of exposure.

The preparation of this monitor chamber makes it possible to evaluate the concentration of a sample actually flowing into the chamber in real time. Therefore, more accurate tests can be conducted.

### Brief Description of Drawings

FIG. 1 is a view showing the first embodiment of a whole smoke exposure apparatus according to the present invention;
FIG. 2A is a view showing a main cross-sectional portion of each module;
FIG. 2B is a left side view of FIG. 2A;
FIG. 2C is a right side view of FIG. 2A;
FIG. 2D is a plan view of FIG. 2A;
FIG. 3 is a plan view of a cell-adhered surface;
FIG. 4 is a view showing the second embodiment of the whole smoke exposure apparatus according to the present invention; and
FIG. 5 is a partial perspective view of a manifold according to the second embodiment.

### Best Mode for Carrying Out the Invention

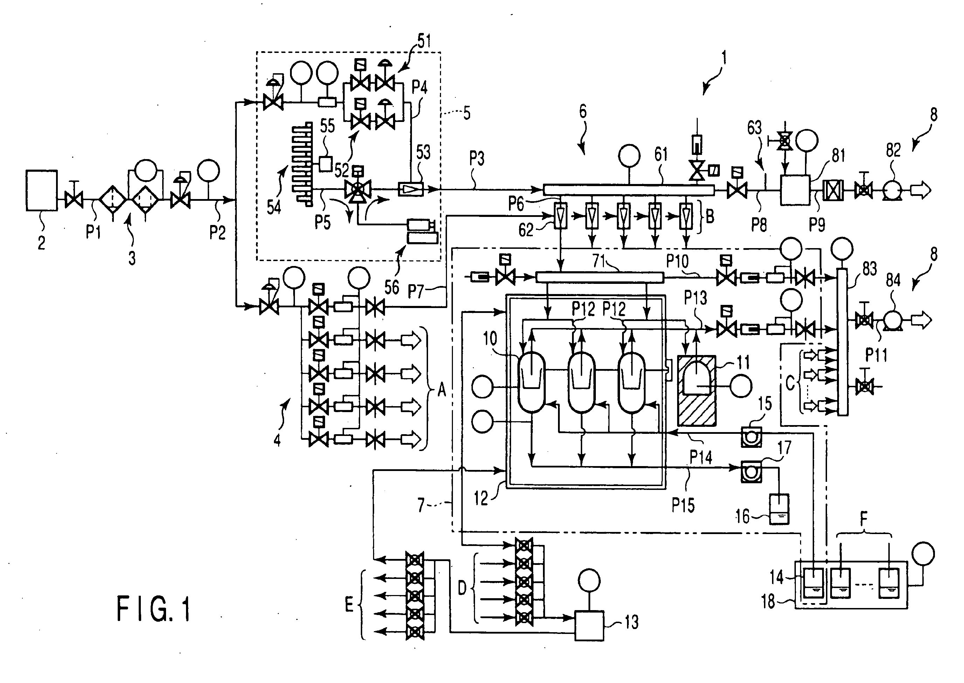

The first embodiment of a whole smoke exposure apparatus according to the present invention will be described in detail below with reference to FIGS. 1 to 3. Referring to FIG. 1, the whole smoke exposure apparatus denoted by reference numeral 1 has a compressor 2. The compressor 2 supplies compressed air to a clean air unit 3 connected thereto through a pipe line P1. The clean air unit 3 is connected to a clean air supply section 4 and smoke emitting section 5 through a pipe line P2 branched into two lines. The smoke emitting section 5 is connected to a dilution means 6 through a pipe line P3. The dilution means 6 is connected to the clean air supply section 4 through a pipe line P7.

The smoke emitting section 5 has a diluent air section 51, clean air section 52, and concentration setting section 53. The diluent air section 51 and clean air section 52 are connected to the two pipe lines branching from the pipe line P2. The pipe lines P2 become a single pipe line P4 again which is connected to the concentration setting section 53. The smoke emitting section 5 has a plurality of tobaccos 54 rotatably supported on a revolver type support tool 55. The tobaccos 54 are connected to the concentration setting section 53 through a cylinder 56 connected to a pipe line P5 in a T shape.

The dilution means 6 is connected to five modules 7 through the same number of pipe lines P6 (FIG. 1 shows only one of the five modules; an illustration of the four remaining modules is omitted), and is connected to an exhaust means 8 through a pipe line P8. The dilution means 6 has a header 61 and concentration setting sections 62 mounted in the respective pipe lines P6. Each of the concentration setting sections 62 may have the same function as that of the concentration setting section 53 mounted in the smoke emitting section 5.

The exhaust means 8 is connected to a first exhaust tank 81 and a vacuum pump 82 connected thereto through a pipe line P9. The first exhaust tank 81 is connected to the header 61 through the pipe line P8. The pipe line P8 is a three-way connector 63 having one line connected to the header 61, another line connected to the first exhaust tank 81, and the remaining line open to the atmosphere.

As described above, the exposure apparatus according to this embodiment is a five-module whole smoke exposure apparatus having five modules. Only one module will be described below. When, however, the apparatus has a plurality of modules as in this embodiment with the five-module structure, all the remaining modules have the same arrangement.

The module 7 has a dilution tank 71 located at an upper position and chambers 10. The dilution tank 71 is connected to the header 61 through the pipe line P6. The dilution tank 71 is connected to a second exhaust tank 83 through a pipe line P10. The second exhaust tank 83 is connected to a second vacuum pump 84 through a pipe line P11.

The whole smoke exposure apparatus 1 according to the first embodiment has the three chambers 10 connected to the dilution tank 71 through pipe lines P12 for each module. That is, the apparatus has a total of 15 chambers (20 chambers if aerosol monitors (to be described later) are added) and five dilution tanks 71 as a whole.

The dilution tank 71 is connected to the two pipe lines P12 having uniform structures. Each of the pipe lines P12 is equally branched into two lines; a total of four branch lines are connected to the chambers 10. The three ends of the four branch lines of the pipe lines P12 are respectively connected to the inlets of the three chambers 10. The one remaining end is connected to the inlet of an aerosol monitor 11 for monitoring the concentration of smoke in the chamber.

The aerosol monitor 11 has the same structure as that of the remaining chambers 10 except that a concentration sensor is mounted in the monitor. The chambers 10, including the aerosol monitor 11, have outlets connected to the second exhaust tank 83 through a pipe line P13, like the dilution tank 71.

The pipe lines P1 to P13 respectively have valves each capable of adjusting the flow rate of a sample or clean air flowing in a corresponding of the pipe lines. The types of valves to be used may be selected in accordance with the application purpose of the respective sections.

The module 7 has a flow path 12 through which isothermal water circulates to keep the temperatures of a culture medium and cell in the chamber 10 constant. The flow path 12 forms a loop running through the module and a first thermostat 13.

The module 7 independently has a culture medium supply tank 14, a tube pump 15 for supplying culture media from the culture medium supply tank 14 to the respective chambers, a culture medium discharge tank 16, and a tube pump 17 for discharging culture media from the respective chambers into the culture medium discharge tank. That is, each chamber 10 in the module 7 has the above two pipe systems.

In the first system, the culture medium supply tank 14 is connected to the supply tube pump 15 through a pipe line, and the tube pump 15 is connected to the three chambers 10 through a pipe line P14 branched into three lines. The culture medium supply tank 14 is housed in a second thermostat 18 to be kept at a constant temperature, preferably about 37°C.

In the second system, in contrast to the first system, discharge pipe lines P15 to which the three chambers 10 are connected are merged into one line at a predetermined position and connected to the discharge tube pump 17. The tube pump 17 is connected to the culture medium discharge tank 16 through a pipe line.

The tube pumps 15, 17 have double-bonded heads.

The apparatus according to this embodiment is a five-module whole smoke exposure apparatus, in which clean area supply sections, dilution means, vacuum pumps, and first and second thermostats are connected to the respective modules through pipe lines equal in number to the modules. Referring to FIG. 1, reference symbols A, B, C, D, E, and F indicate that the four omitted portions have the same structure as that of one portion described in detail.

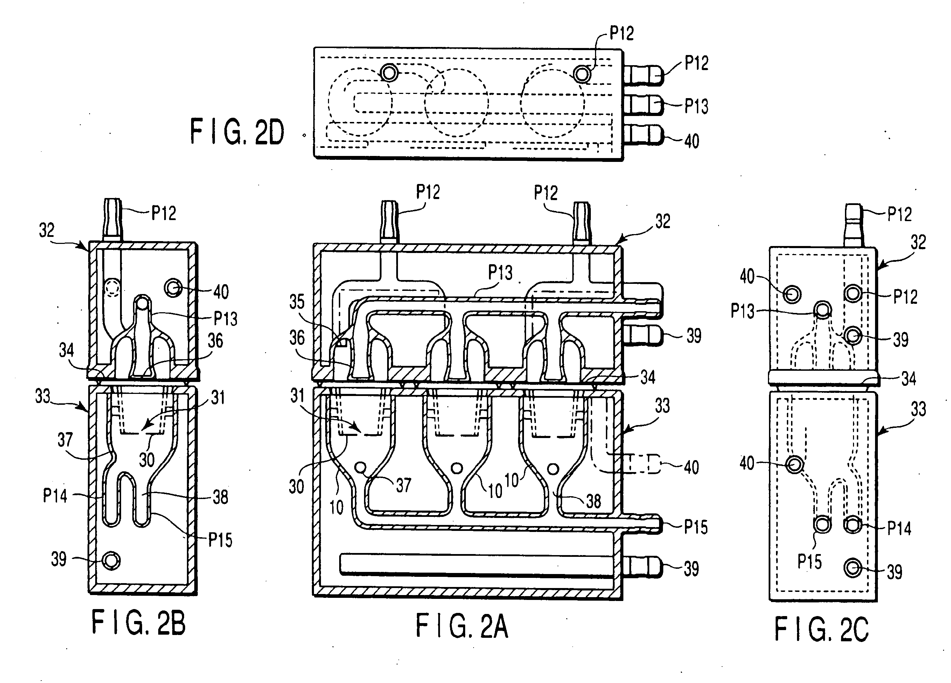

FIGS. 2A to 2D are views showing the details of the main part of the module in the first embodiment shown in FIG. 1. As shown in FIG. 1, the module 7 has the three chambers 10. Each of these chambers has an almost cylindrical shape with a lower portion tapering off in a conical shape. These chambers incorporate cell-adhered surfaces 30 to which cells are adhered. Each of the three chambers 10 is comprised of an upper unit 32 and lower unit 33. A seal member 34 serving as a blocking means for hermetically sealing the lower unit 33 is provided between the upper unit 32 and the lower unit 33. The seal member 34 is preferably made of a sterilizable material.

The upper unit 32 has a sample inlet 35 and outlet 36. The lower unit 33 has a culture medium inlet 37 and outlet 38.

The inlet 35 is formed in the peripheral portion of the upper surface of the chamber 10 in the radial direction so as to introduce smoke supplied from the dilution tank. The pipe line P12 is connected to the inlet 35. The pipe line P13 having one end formed into the outlet 36 open to the inside of the chamber extends through almost the center of the upper surface of chamber. That is, the outlet 36 is provided near the cell-adhered surface 30 inside the chamber.

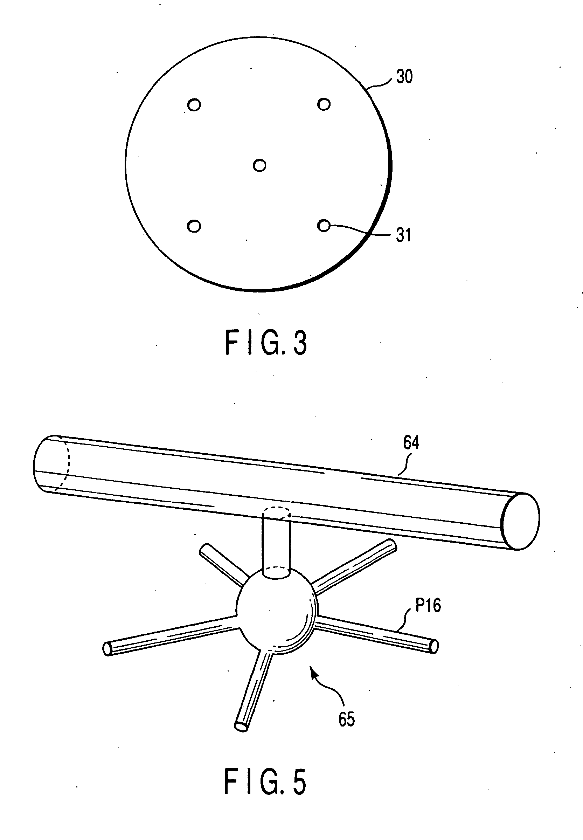

The cell-adhered surface 30 is a membrane made of polyethylene terephthalate (PET), and has a plurality of holes 31 through which the culture medium supplied into the chamber 10 passes. In this embodiment, as shown in FIG. 3, the holes 31 are respectively located at the center of the cell-adhered surface 30 and the upper, lower, left, and right positions surrounding the center. The material for the cell-adhered surface 30, the number of holes 31, and the positions of the holes 31 are not limited to those in the above arrangement.

The inlet 37 for the culture medium supplied from the culture medium supply tank 14 through the pipe line P14 is formed in the conical inclined surface portion of the lower portion of each chamber 10. The outlet 38 is formed in a portion corresponding to the vertex of this cone (i.e., near the bottom portion of the chamber 10). The pipe line P15 is connected to the outlet 38. The inlet 37 located above the outlet 38.

The operation of the first embodiment of the whole smoke exposure apparatus 1 having the above embodiment will be described next. Smoke as a sample is sequentially emitted from the tobaccos 54 supported on the revolver type support tool 55 of the smoke emitting section 5. Smoke can be consecutively emitted by the amount corresponding to the number of tobaccos 54 by sequentially rotating the support tool 55. The smoke emitted from the tobacco 54 is collected in the cylinder 56 through the pipe line P5. The smoke collected in the cylinder 56 is supplied to the concentration setting section 53, which performs concentration adjustment (primary dilution) using clean air supplied from the diluent air section 51. The primarily diluted smoke is temporarily stored in the header 61 of the dilution means 6 through the pipe line P3. The inside of the above pipe line though which smoke flows is cleaned at predetermined intervals under the control of the clean air section 52.

The smoke is temporarily stored in the header 61 and supplied to the concentration setting sections 62 through the pipe lines P6. The concentration setting sections 62 are connected to the clean air supply section 4 through the pipe lines P7 respectively connected to the pipe lines P6 in a T shape. The concentration of the smoke stored in the header 61 is adjusted (secondary dilution) by using clean air supplied from the clean air supply section 4 to the respective pipe lines P6. With this operation, since the smoke is temporarily stored in the header 61 and hardly escapes outside, the absolute amount of smoke suffers a little loss at the time of dilution.

In addition, the sample in the header 61 is held in the exhaust tank 81 through the pipe line P8, and can be exhausted by the vacuum pump 82, as needed. The pipe line P8 is the three-way connector 63 having one line open to the atmosphere, and hence prevents the occurrence of a negative pressure between the vacuum pump 82 and the header 61 as the vacuum pump pressure decreases. This makes it possible to reduce pressure fluctuations in each pipe line described above.

The smoke secondarily diluted by the dilution means can be supplied to the dilution tank 71 placed above each module 7 after being set at a predetermined concentration. When there are a plurality of modules 7, smoke supplied to the dilution tanks 71 placed above the respective modules 7 may be set at different concentrations.

The smoke stored in the dilution tank 71 is introduced into the respective chambers 10 from the inlets 35 formed in the upper surfaces of the chambers 10 through the uniformly branched pipe line P12. At this time, the diluted smoke is temporarily stored in the dilution tank 71, and the pipe lines P12 are uniformly provided for the respective chambers. This structure makes it possible to supply homogeneous smoke into the respective chambers 10. Therefore, a high-precision exposure test can be conducted.

When smoke is introduced, the cell-adhered surface 30 has been immersed in the culture medium supplied into each chamber 10 and hence the cell is protected by the culture medium. After smoke is introduced, the fluid level of the culture medium is lowered to a level lower than the cell-adhered surface 30. When the cell is exposed to the smoke, the entrance/exit of smoke to/from the module 7 is shut off, and the lower unit 33 of the chamber is hermetically sealed by the seal member 34. By maintaining this state, the smoke settles down in the chamber 10 for a predetermined period of time, and the cell is exposed to the smoke.

As described above, the cell is protected by the culture medium when a sample is introduced, and hence is free from the influence of the blowing pressure of the sample. In addition, since the module is shut off from the outside at the time of exposure to the smoke, the sample and cells are not influenced by the flow of clean air and the like. That is, in an exposure test by this apparatus, introduction of a sample into the chamber 10 and exposure of a cell to the sample are performed as different steps, and each chamber 10 is shut off from the outside at the time of exposure and functions as a sample reservoir tank.

In addition, the flow of air in each chamber 10 is blocked at the time of exposure to smoke, and the smoke settles down. For this reason, a cell can be reliably exposed to even smoke containing a particulate phase as well as a vapor phase. For the same reason, the efficiency of exposure of a cell to smoke using this apparatus is very high.

In addition, the seal member 34 is formed from a sterile member, and hence contamination of the culture medium or cell-adhered surface due to fungi can be prevented.

Each chamber has a function of measuring and controlling a pressure even at the time of exposure to smoke. This makes it possible to prevent pressure changes occurring in each operation from influencing the cell.

Isothermal water is supplied from the first thermostat 13 into the module 7 through an inlet 39 and circulates along the flow path 12 for isothermal water in the module 7 to thermally insulate the cell-adhered surface 30 and culture medium. This isothermal water flows out of the module through an outlet 40 and returns to the first thermostat 13. This isothermal water is preferably set to keep the cell-adhered surface 30 and culture medium at about 37°C. The isothermal water may be another kind of isothermal medium.

After exposure of the cell to the smoke, the smoke in each chamber is exhausted out of the module through the outlet 36 formed in the center of the upper surface of the chamber and the pipe line P13. In this case, as described above, since the outlet 36 is formed near the cell-adhered surface 30, the exhaust efficiency of the sample after sedimentation/exposure is high.

Excess part of the sample or clean air in each chamber and the dilution tank 71 is stored in the second exhaust tank 83 through the pipe lines P10 and P13. The stored extra part of the sample or clean air can be exhausted as needed.

One exposure test is completed through a series of operations from emission of smoke to exhaustion of smoke in the above manner. Such a series of operations can be consecutively performed in accordance with the number of tobaccos 54 held by the revolver type support tool 55 described above.

Upon completion of one exposure test, the culture medium is replaced with a new, fresh culture medium through the inlet 37 and outlet 38. As shown in FIG. 1, the new culture medium is retained in the culture medium supply tank 14. The tank 14 is installed in the second thermostat 18, and the culture medium is preferably held at about 37°C.

The culture medium is delivered from the culture medium supply tank 14 into the module 7 by the tube pump 15 and supplied into each chamber 10 through the inlet 37 in the module 7. As described above, at the time of introduction of smoke, a cell is immersed in the culture medium to be protected, and the fluid level of the culture medium is lowered to a level lower than the cell-adhered surface 30 at the time of exposure to the smoke. The raising and lowering of the fluid level of each culture medium can be controlled by a sensor installed for each module 7. In addition, the fluid level of a culture medium can be quickly raised or lowered by letting the culture medium pass through the holes 31 formed in the cell-adhered surface 30. The replacement speed of the culture media is set to a predetermined speed high enough to prevent any adverse effect on cells.

The culture medium in which a soluble component in smoke has dissolved at the time of introduction of the smoke and at the time of exposure to the smoke is discharged from the outlet 38 formed in the lower portion of each chamber 10 by the tube pump 17 and delivered to the culture medium discharge tank 16 through the pipe line P15.

Preferably, since the outlet 38 is formed independently of the culture medium inlet 37, the culture medium in which a soluble component in a sample has dissolved at the time of exposure can be completely discharged, and a new culture medium can always be supplied. That is, no culture medium containing a soluble component in smoke is mixed with a newly supplied culture medium. In addition, since two pump heads are contact-bonded to the tube pumps 15 and 17, leakage of a culture medium from piping tubes can be prevented.

In the first embodiment, the inlet 37 is formed above the outlet 38, and the lower portion of the chamber 10 tapers off and inclines toward the outlet 38. This makes it possible to quickly and efficiently replace the culture medium in each chamber 10.

As described above, the holes 31 formed in the cell-adhered surface 30 allow the fluid level of a culture medium to be quickly raised and lowered, and the structure of each chamber 10 makes it possible to quickly supply and discharge a culture medium. Replacement of culture media can therefore be done while a sample is blown. Consequently, the chambers 10 of this apparatus allow quick, efficient, consecutive exposure tests.

Since the module 7 includes at least one aerosol monitor 11 serving as a monitor chamber having a concentration measurement sensor for measuring the concentration of smoke in each chamber 10 at the time of exposure to the smoke, real-time monitoring in each chamber 10 during exposure to the smoke can be done. This makes it possible to evaluate an actual exposure amount.

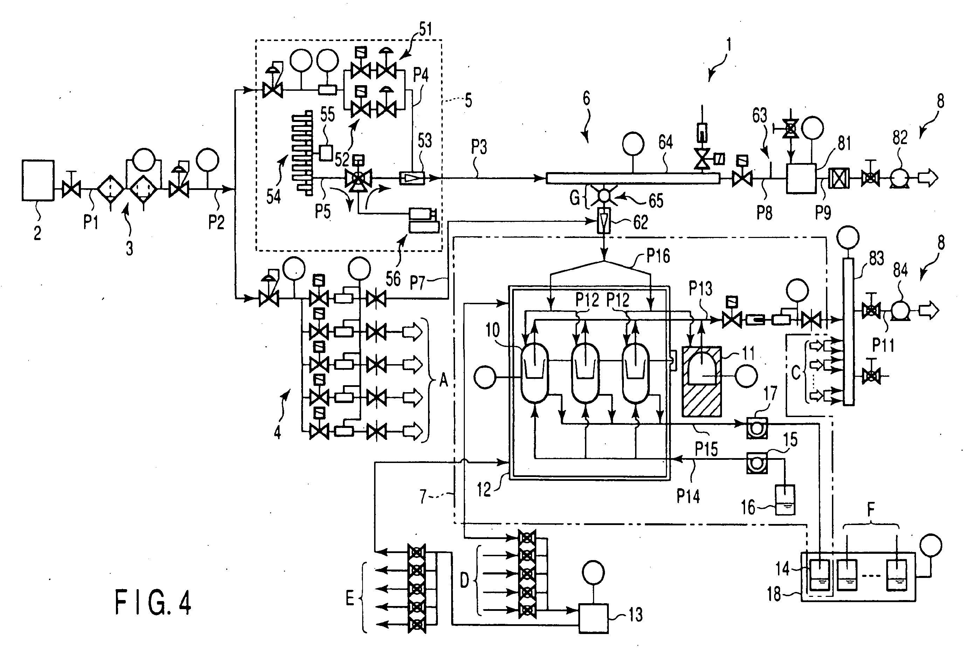

The second embodiment of the whole smoke exposure apparatus according to the present invention will be described in detail below with reference to FIGS. 4 and 5. As shown in FIG. 4, the basic arrangement and operation of the whole smoke exposure apparatus according to this embodiment are the same as those of the first embodiment. Only the different arrangement from the first embodiment will be described below.

In the second embodiment, the dilution means 6 includes a header 64 which temporarily stores the smoke emitted by the smoke emitting section 5, a manifold 65 which is connected to the header 64 through a single pipe line, and a plurality of concentration setting sections 62 which are uniformly connected to the manifold 65. As shown in FIG. 5, the manifold 65 has five pipe lines P16 which are uniformly branched therefrom. The concentration setting sections 62 are respectively provided for the pipe lines P16. The pipe lines P16 are respectively connected to modules 7. Each pipe line P16 is branched into two lines in the module 7. Each of the two pipe lines is further branched into two pipe lines to form four pipe lines. Three ends of the four branch pipe lines of the pipe line P16 are respectively connected to the inlets of three chambers 10. The one remaining end is connected to the inlet of an aerosol monitor 11 for monitoring the concentration of smoke in the chamber. In accordance with this structure, the dilution tank 71 provided in the first embodiment is removed. Referring to FIG. 4, reference symbol G indicates that the four omitted portions have the same structure as that of one portion described in detail.

In the second embodiment, the culture medium supply and discharge directions are opposite to those in the first embodiment. More specifically, the chamber 10 includes a culture medium supply tank 14 connected to an inlet positioned at the bottom portion of the chamber 10, and a culture medium discharge tank 16 connected to an outlet positioned above the inlet. In correspondence with the arrangement of the chamber 10, a tank (the culture medium supply tank 14 in the second embodiment) connected to the bottom portion of the chamber 10 is housed in the second thermostat 18 and is preferably held at 37°C.

The operation of the second embodiment having the above arrangement is basically the same as that of the first embodiment. The smoke emitted by the smoke emitting section 5 is temporarily stored in the header 64 through a pipe line P3. The smoke temporarily stored in the header 64 is supplied to the manifold 65 through a pipe line. This smoke can be further supplied to the module 7 after a concentration is set by a plurality of concentration setting sections 63 uniformly connected to the manifold 65. If there are a plurality of modules, smoke can be supplied to the respective modules 7 after being set at different concentrations. The smoke is delivered to the chambers 10 connected through the four branched pipe lines P16 in the module 7. A culture medium is supplied into the chamber 10 through the inlet positioned at the bottom portion of the chamber 10, and discharged from the outlet located above the inlet.

In the second embodiment, the smoke emitted by the smoke emitting section 5 can flow into a plurality of chambers 10, as samples uniform in concentration and amount, from the manifold 65 connected to the header 64 through the concentration setting section 63. This allows a high-precision exposure test in a whole smoke exposure apparatus 1 having a plurality of chambers 10, and increases the exposure efficiency. In addition, the installation of the manifold 65 between the header 64 and the concentration setting section 63 makes it possible to stabilize the behavior of diluted smoke and introduce homogenous smoke into the chambers 10.

A culture medium is supplied into the chamber 10 through an inlet positioned at the bottom portion of the chamber, and is discharged through an outlet positioned above the inlet. This can prevent the occurrence of air bubbles in a culture medium.

Other operations and effects in the second embodiment are the same as those in the first embodiment.

The above modules, chambers, and remaining constituent elements described above are the same in number and shape as those in the first embodiment of the whole smoke exposure apparatus according to the present invention, and are not limited in a narrow sense as well as they fall within the technical idea and the scope of claims of the present invention.

### Industrial Applicability

As described above, in the whole smoke exposure apparatus according to the present invention, since the flow of smoke into the chamber is blocked at the time of exposure, the smoke with a set concentration comes into contact with a cell-adhered surface in a sealed state without changing the set concentration. This allows the cell to be reliably exposed to smoke containing a particulate phase as well as a vapor phase or their mixture. In addition, the cell is free from the influence of the blowing pressure of a sample or the flow of clean air. That is, efficient, high-precision exposure tests can be conducted by the whole smoke exposure apparatus according to the present invention.

## Claims

1. A whole smoke exposure apparatus (1) comprising:
a smoke emitting section (5) which emits smoke containing particulate matter;
a dilution means (6) for diluting the smoke emitted by the smoke emitting section (5) by adding a gas to the smoke;
an air unit (3) which supplies the gas to said dilution means (6);
at least one exposure chamber (10) which has a cell-adhered surface (30) therein and into which the smoke diluted by the dilution means (6) is introduced;
a blocking means for shutting off a gas flow so as to seal the chamber (10) after the smoke is introduced into the chamber (10);
a culture medium supply tank (14), a tube pump (15), a pipe line connecting the culture medium supply tank (14) to the tube pump (15) and a pipe line (P14) connecting the tube pump (15) to the at least one exposure chamber (10) for supplying a culture medium in said at least one exposure chamber (10) so that the cell-adhered surface (30) is immersed in the culture medium to protect a cell attached to the cell-adhered surface (30) when the smoke is introduced into the exposure chamber (10);
a culture medium discharge tank (16), a discharge tube pump (17), a pipe line connecting the culture medium discharge tank (16) to the discharge tube pump (17) and a discharge pipe line (P15) connecting the discharge tube pump (17) to the at least one exposure chamber (10) for lowering a level of the culture medium lower than the cell-adhered surface (30) after the smoke is introduced into the chamber (10), and an exhaust means (8) for exhausting the smoke introduced into the chamber (10).

2. A whole smoke exposure apparatus according to claim 1, wherein the dilution means (6) includes a concentration setting means (62) for arbitrarily setting a concentration of a smoke to be diluted.

3. A whole smoke exposure apparatus according to claim 1 or 2, further comprising a header (61) which is located between the smoke emitting section (5) and the chamber (10) and in which the smoke emitted by the smoke emitting section (5) is temporarily stored before being introduced into the chamber (10).

4. A whole smoke exposure apparatus according to claim 3, further comprising a three-way connector (63) having one pipe line connected to the header (61), another pipe line connected to the exhaust means (8), and still another pipe line open to the atmosphere.

5. A whole smoke exposure apparatus according to any one of claims 1 to 4, further comprising a manifold (65) which is positioned between the smoke emitting section (5) and a plurality of chambers (10) for exposure, and has a piping structure uniformly branched to allow the smoke emitted by the smoke emitting section (5) to be introduced to be supplied to said plurality of chambers (10) for exposure.

6. A whole smoke exposure apparatus according to any one of claims 1 to 5, further comprising a dilution tank (71) which is positioned between the smoke emitting section (5) and a plurality of chambers (10) for exposure to allow smoke diluted by the dilution means (6) to flow into the dilution tank (71), and has a piping structure uniformly connected to said plurality of chambers (10) for exposure.

7. A whole smoke exposure apparatus according to any one claims 1 to 6, wherein the chamber (10) has an inlet (35) for the smoke which is positioned at an upper portion (32) of the chamber (10), and an outlet (36) for the smoke which is positioned near the cell-adhered surface (30).

8. A whole smoke exposure apparatus according to any one of claims 1 to 7, wherein the chamber (10) has a structure (33) which has an inlet (37) and outlet (38) for a culture medium in which the cell-adhered surface (30) is to be immersed, and in which the culture medium inlet (37) and outlet (38) are provided at different positions.

9. A whole smoke exposure apparatus according to claim 8, wherein the cell-adhered surface (30) has at least one hole (31) through which the culture medium passes.

10. A whole smoke exposure apparatus according to claim 8 or 9, further comprising a warm keeping means for keeping the culture medium and/or the cell-adhered surface (30) at a constant temperature.

11. A whole smoke exposure apparatus according to any one of claims 1 to 10, further comprising a means for measuring and controlling a pressure in the chamber.

12. A whole smoke exposure apparatus according to any one of claims 1 to 11, further comprising at least one monitor chamber (11) which has concentration measurement sensor for measuring a concentration of the smoke in the chamber (10) at the time of exposure.

## Patentansprüche

1. Komplette Rauch-Aussetzungseinichtung (1), aufweisend:
einen Rauch abgebenden Abschnitt (5), der Schwebstoffe enthaltenden Rauch abgibt;
- Verdünnungsmittel (6) zum Verdünnen des von dem Rauch abgebenden Abschnitt (5) abgegebenen Rauchs durch Hinzuführen eines Gases zum Rauch;
- eine Lufteinheit (3), die den Verdünnungsmitteln (6) das Gas zuführt;
- mindestens eine Aussetzungskammer (10), die eine von Zellen behaftete Oberfläche (30) enthält und in die der durch die Verdünnungsmittel (6) verdünnte Rauch eingeführt wird;
- Blockiermittel zum Absperren eines Gasstroms, um die Kammer (10) zu verschließen, nachdem der Rauch in die Kammer (10) eingeführt wurde;
- einen Kultursubstrat-Zuführtank (14), eine Rohrpumpe (15), eine Rohrleitung, die den Kultursubstrat-Zuführtank mit der Rohrpumpe (15) verbindet, und eine Rohrleitung (P14), die die Rohrpumpe (15) mit der mindestens einen Aussetzungskammer (10) verbindet, um der mindestens einen Aussetzungskammer (10) ein Kultursubstrat zuzuführen, so dass die von Zellen behaftete Oberfläche (30) in das Kultursubstrat eingetaucht wird, um eine an der von Zellen behafteten Oberfläche (30) haftende Zelle zu schützen, wenn der Rauch in die Aussetzungskammer (10) eingeführt wird;
- einen Kultursubstrat-Ausgabetank (16), eine Rohraustragspumpe (17), eine Rohrleitung, die den Kultursubstrat-Ausgabetank (16) mit der Rohraustragspumpe (17) verbindet, und eine Rohraustragsleitung (P15), die die Rohraustragspumpe (17) mit der mindestens einen Aussetzungskammer (10) verbindet, um einen Stand des Kultursubstrats auf einen Stand unterhalb der von Zellen behafteten Oberfläche (30) zu senken, nachdem der Rauch in die Kammer (10) eingeführt wurde, und Ausstoßmittel (8) zum Ausstoßen des in die Kammer (10) eingeführten Rauchs.

2. Komplette Rauch-Aussetzungseinrichtung nach Anspruch 1, wobei die Verdünnungsmittel (6) Konzentrationseinstellmittel (62) zum beliebigen Einstellen einer Konzentration von zu verdünnendem Rauch umfassen.

3. Komplette Rauch-Aussetzungseinrichtung nach Anspruch 1 oder 2, ferner aufweisend einen Sammler (61), der sich zwischen dem Rauch abgebenden Abschnitt (5) und der Kammer (10) befindet und in der der von dem Rauch abgebenden Abschnitt (5) abgegebene Rauch vorübergehend gelagert wird, bevor er in die Kammer (10) eingeführt wird.

4. Komplette Rauch-Aussetzungseinrichtung nach Anspruch 3, ferner aufweisend ein Dreiwege-Verbindungsstück (63) mit einer Rohrleitung, die mit dem Sammler (61) verbunden ist, einer weiteren Rohrleitung, die mit den Aussetzungsmitteln (8) verbunden ist, und noch einer weiteren Rohrleitung, die zur Atmosphäre geöffnet ist.

5. Komplette Rauch-Aussetzungseinrichtung nach einem der Ansprüche 1 bis 4, ferner aufweisend einen Verteiler (65), der zwischen dem Rauch abgebenden Abschnitt (5) und einer Vielzahl von Kammern (10) zum Aussetzen positioniert ist, und der eine Rohrleitungsstruktur aufweist, die gleichmäßig verzweigt ist, um zu ermöglichen, dass der von dem Rauch abgebenden Abschnitt (5) abgegebene Rauch eingeführt wird, um der Vielzahl von Kammern (10) zum Aussetzen zugeführt zu werden.

6. Komplette Rauch-Aussetzungseinrichtung nach einem der Ansprüche 1 bis 5, ferner aufweisend einen Verdünnungstank (71), der zwischen dem Rauch abgebenden Abschnitt (5) und einer Vielzahl von Kammern (10) zum Aussetzen positioniert ist, um zu ermöglichen, dass von den Verdünnungsmitteln (6) verdünnter Rauch in den Verdünnungstank (71) fließen kann, und der eine Rohrleitungsstruktur aufweist, die gleichmäßig mit der Vielzahl von Kammern (10) zum Aussetzen verbunden ist.

7. Komplette Rauch-Aussetzungseinrichtung nach einem der Ansprüche 1 bis 6, wobei die Kammer (10) einen Einlass (35) für den Rauch, der an einem oberen Abschnitt (32) der Kammer (10) positioniert ist, und einen Auslass (36) für den Rauch aufweist, der nahe der von Zellen behafteten Oberfläche (30) positioniert ist.

8. Komplette Rauch-Aussetzungseinrichtung nach einem der Ansprüche 1 bis 7, wobei die Kammer (10) eine Struktur (33) aufweist, die einen Einlass (37) und einen Auslass (38) für ein Kultursubstrat aufweist, in das die von Zellen behaftete Oberfläche (30) einzutauchen ist, und in der der Kultursubstrat-Einlass (37) und -Auslass (38) an verschiedenen Positionen vorgesehen sind.

9. Komplette Rauch-Aussetzungseinrichtung nach Anspruch 8, wobei die von Zellen behaftete Oberfläche (30) mindestens ein Loch (31) aufweist, durch die das Kultursubstrat hindurchgeht.

10. Komplette Rauch-Aussetzungseinrichtung nach Anspruch 8 oder 9, ferner aufweisend Warmhaltemittel zum Halten des Kultursubstrats und/oder der von Zellen behafteten Oberfläche (30) auf einer konstanten Temperatur.

11. Komplette Rauch-Aussetzungseinrichtung nach einem der Ansprüche 1 bis 10, ferner aufweisend Mittel zum Messen und Steuern eines Drucks in der Kammer.

12. Komplette Rauch-Aussetzungseinrichtung nach einem der Ansprüche 1 bis 11, ferner aufweisend mindestens eine Überwachungskammer (11), die einen Konzentrationsmesssensor zum Messen einer Konzentration des Rauchs in der Kammer (10) zum Zeitpunkt des Aussetzens aufweist.

## Revendications

1. Appareil d'exposition à la fumée complète (1) comportant :
une section d'émission de fumée (5) qui émet de la fumée contenant de la matière en particules ;
des moyens de dilution (6) destinés à diluer de la fumée émise par la section d'émission de fumée (5) en ajoutant un gaz à la fumée ;
une unité d'air (3) qui délivre le gaz aux dits moyens de dilution (5)
au moins une chambre d'exposition (10) qui a une surface à cellule collée (30) à l'intérieur et dans laquelle la fumée diluée par les moyens de dilution (6) est introduite ;
des moyens de blocage destinés à arrêter un écoulement de gaz de façon à obturer la chambre (10) une fois que la fumée est introduite dans la chambre (10) ;
un réservoir d'alimentation en milieu de culture (14), une pompe à tube (15), une ligne de tuyau reliant le réservoir d'alimentation en milieu de culture (14) à la pompe à tube (15) et une ligne de tuyau (P14) reliant la pompe à tube (15) à la au moins une chambre d'exposition (10) afin de délivrer un milieu de culture dans ladite au moins une chambre d'exposition (10) de telle sorte que la surface à cellule collée (30) est immergée dans le milieu de culture afin de protéger une cellule fixée sur la surface à cellule collée (30) quand la fumée est introduite dans la chambre d'exposition (10) ;
un réservoir d'évacuation de milieu de culture (16), une pompe à tube d'évacuation (17), une ligne de tuyau reliant le réservoir d'évacuation de milieu de culture (16) à la
pompe à tube d'évacuation (17) et une ligne de tuyau d'évacuation (P15) reliant la pompe à tube d'évacuation (17) à la au moins une chambre d'exposition (10) afin d'abaisser un niveau du milieu de culture en dessous de la surface à cellule collée (30) une fois que la fumée est introduite dans la chambre (10), et des moyens d'échappement (8) pour l'échappement de la fumée introduite dans la chambre (10).

2. Appareil d'exposition à la fumée complète selon la revendication 1, dans lequel les moyens de dilution (6) comprennent des moyens de détermination de concentration (62) destinés à déterminer arbitrairement une concentration d'une fumée devant être diluée.

3. Appareil d'exposition à la fumée complète selon la revendication 1 ou 2, comportant en outre un collecteur (61) qui est disposé entre la section d'émission de fumée (5) et la chambre (10) et dans lequel la fumée émise par la section d'émission de fumée (5) est temporairement stockée avant d'être introduite dans la chambre (10).

4. Appareil d'exposition à la fumée complète selon la revendication 3, comportant en outre un connecteur à trois voies (63) ayant une ligne de tuyau reliée au collecteur (61), une autre ligne de tuyau reliée aux moyens d'échappement (8), 1 et encore une autre ligne de tuyau ouverte sur l'atmosphère.

5. Appareil d'exposition à la fumée complète selon l'une quelconque des revendications 1 à 4, comportant en outre une tubulure (65) qui est positionnée entre la section d'émission de fumée (5) et une pluralité de chambres (10) pour exposition, et a une structure de tuyauterie uniformément raccordée pour permettre à la fumée émise par la section d'émission de fumée (5) d'être introduite afin d'être délivrée à ladite pluralité de chambres (10) pour exposition.

6. Appareil d'exposition à la fumée complète selon l'une quelconque des revendications 1 à 5, comportant en outre un réservoir de dilution (71) qui est positionné entre la section d'émission de fumée (5) et une pluralité de chambres (10) pour exposition afin de permettre à de la fumée diluée par les moyens de dilution (6) de s'écouler dans le réservoir de dilution (71), et a une structure de tuyauterie reliée uniformément à ladite pluralité de chambres (10) pour exposition.

7. Appareil d'exposition à la fumée complète selon l'une quelconque des revendications 1 à 6, dans lequel la chambre (10) a une entrée (35) pour la fumée qui est positionnée au niveau d'une partie supérieure (32) de la chambre (10), et une sortie (36) pour la fumée qui est positionnée près de la surface à cellule collée (30).

8. Appareil d'exposition à la fumée complète selon l'une quelconque des revendications 1 à 7, dans lequel la chambre (10) a une structure (33) qui a une entrée (37) et une sortie (38) pour un milieu de culture dans lequel la surface à cellule collée (30) doit être immergée, et dans lequel l'entrée de milieu de culture (37) et la sortie (38) sont prévus dans des positions différentes.

9. Appareil d'exposition à la fumée complète selon la revendication 8, dans lequel la surface à cellule collée (30) a au moins un trou (31) à travers lequel le milieu de culture passe.

10. Appareil d'exposition à la fumée complète selon la revendication 8 ou 9, comportant en outre des moyens de maintien au chaud destinés à maintenir le milieu de culture et/ou la surface à cellule collée (30) à une température constante.

11. Appareil d'exposition à la fumée complète selon l'une quelconque des revendications 1 à 10, comportant en outre des moyens destinés à mesurer et commander une pression dans la chambre.

12. Appareil d'exposition à la fumée complète selon l'une quelconque des revendications 1 à 11, comportant en outre au moins une chambre de contrôle (11) qui a un capteur de mesure de concentration destiné à mesurer une concentration de la fumée dans la chambre (10) au moment de l'exposition.
